# EUROPEAN PATENT APPLICATION

(11) **EP 4 282 949 A1**
(43) Date of publication of application: **29.11.2023**
(21) Application number: 21897244.6
(22) Date of filing: 12.11.2021
(51) Int. Cl.: C12N 1/20, C12R 1/38

(54) **PSEUDOMONAS PALMENSIS**

(30) Priority: 26.11.2020 ES 202031185
(71) Applicant: Biobab R&D, S.L., 35260 Agüimes (Las Palmas) (ES)
(72) Inventor: GUTIERREZ ALBANCHEZ, Enrique, 28925 Alcorcón (Madrid) (ES); HORCHE TRUEBA, Ignacio, 28925 Alcorcón (Madrid) (ES); LUCAS GARCÍA, Jose Antonio, 28760 Tres Cantos (Madrid) (ES); RAMOS SOLANO, Beatriz, 28224 Pozuelo de Alarcon (ES); GUTIERREZ MAÑERO, Francisco Javier, 28691 Villanueva de la Cañada (Madrid) (ES)
(86) International application number: PCT/ES2021/070820
(87) International publication number: WO 2022/112631

(57) **Abstract**

Bacterial strain Pseudomonas palmensis BBB001, microorganism of the gram-negative group of bacteria, genus Pseudomonas , stimulator of plant adaptive metabolism against abiotic stress and enhancer of plant nutrition in iron and other nutrients. This strain, isolated from the rhizosphere of Nicotiana glauca, has been characterised from a morphological, biochemical and genetic standpoint by whole-genome sequencing, being identified as a new species, reduces oxidative stress in plants, inducing higher production and improved adaptation to abiotic stress conditions; for example, due to lack of water or high salinity, such that it can be used, alone or in combination with other strains, to increase plant fitness in plant species of agronomic food interest. Furthermore, since it is capable of enhancing iron, phosphorus and potassium nutrition, it can also be used as a biofertiliser, both in organic and conventional agriculture.

## Description

The current invention concerns the bacterial strain Pseudomonas palmensis (BBB001 internal laboratory code), as a new species of the genus Pseudomonas, for use in the treatment of plants to improve adaptation to abiotic stress, decreasing oxidative stress in order to increase growth and yield. In addition, it is able to produce siderophores, improving iron nutrition of plants, reversing iron deficiency symptoms, and to stimulate the mobilisation of other nutrients such as phosphorus and potassium, so it is also of interest as a biofertiliser for organic and conventional agriculture.

This strain has been deposited for patent purposes in the Spanish Type Culture Collection of the University of Valencia (CECT), where the number 30222 has been assigned to it.

### TECHNICAL FIELD. -

The invention falls within the field of agri-food biotechnology, specifically within plant growth regulating bacteria, since the aforementioned bacterial strain can be used as the basis for the preparation of different types of products that stimulate the adaptive metabolism of plants. These products will improve plant adaptation to different abiotic stress conditions such as water deficit or excess soil salinity.

Also within the field of organic biofertilisers, as the strain can be used to improve plant nutrition in general and more specifically, iron uptake in soils, especially with basic pH, both in any bacterial formulation and with bacterial metabolites obtained under certain conditions.

Furthermore, since it is a strain belonging to a new species, following the terminology used for the indexing of bacteria in Bergey's "Handbook of Deterministic Bacteriology", March 2001 edition, together with the genomic analysis performed, the invention can be classified as one of the bacterial species of the genus Pseudomonas.

### STATE OF THE ART.-

The mechanisms of action of plant growth-promoting bacteria can be summarised in two types: direct, when the bacteria or their metabolites alter the plant's metabolism (hormonal activity, stimulation of adaptive mechanisms), and indirect, when they synthesise compounds that facilitate the uptake or mobilisation of nutrients or prevent the growth of pathogenic micro-organisms on the plant, without altering the plant's metabolism. In the case of this patent, both are of interest. The plant has a secondary metabolism, highly inducible, related to the adaptation of the plant to adverse situations it has to face during its life.

The genus Pseudomonas is common among soil bacteria. According to the taxonomy of Bergey's Manual, March 2001 edition, this bacterium is included in the Domain Bacteria, Phylum Proteobacteria, Class gamma proteobacteria, Order Pseudomonadales, Family Pseudomonaceae. The genus Pseudomonas is very common in the soil system, and has been repeatedly described as a protective bacterium against various plant diseases. Some bacteria of this group produce fluorescent yellow-green pigments that are easily soluble in water. Among other functions, these pigments act as siderophores (molecules capable of capturing iron from the medium for the microorganism's metabolism). In addition, they show great metabolic versatility due to a large number of plasmids containing inducible operons for the synthesis of specific enzymes that allow the catabolisation of compounds present in the medium. The presence of Pseudomonas sp. in the rhizosphere of different plants beneficially affects their physiology indicating that its selection by the plant at the rhizospheric level is highly possible. The effects of this strain on the physiology of different plants indicate that the plant selects them for its benefit.

There are numerous references in literature concerning compositions and formulations containing strains of the genus Pseudomonas, alone or in combination with other organisms to stimulate and improve plant growth, or that serve as a basis for the formulation of phytosanitary products, protectors against pathogens or to preserve the good growth of crops, some of which have been patented, European patent with publication numbers in Spain ES2707807-T3, 2019-04-05, New fluorescent Pseudomonas fluorescens of the species Pseudomonas azotoformans for the stimulation of the emergence and growth of plants, and ES2624788-T3, 2017-07-17, Biological culture of a strain of the species Pseudomonas graminis and use of this culture as an antagonist for the biological control of pathogenic bacteria; or national patent ES2572746-B1, 2016-09-21, for Procedure for obtaining a plant growth promoting composition comprising Pseudomonas putida and uses thereof.

In this patent application, new technologies have made it possible to identify a new species, Pseudomonas palmensis BBB001, based on its unique genetic characteristics, which differentiate it from other Pseudomonas strains when the genome of BBB001 is compared to the 254 complete and available genomes of Pseudomonas.

The parameters that allow the identification of a new species are any of the following: i) average nucleotide identity (ANI) and average amino acid identity (AAI), with threshold values to define a new species below 95%; ii) DNA-DNA digital hybridisation (dDDH), with values below 70%; iii) guanine-cytosine content (G+C), values above 1%; iv) tetranucleotide usage frequency correlation index (TETRA), with values below 0. 99; v) multilocus sequence analysis (MLSA), with values below 97%.

Comparing the BBB001 genome with the 254 available genomes, Pseudomonas alkylphenolica is the closest species as ANI and AAI values were 83.39% and 85.11%, respectively, both below 95%, DNA-DNA digital hybridisation (dDDH) values 27.10%, below 70% and guanine-cytosine (G+C) content 3.43%, above 1%. The tetranucleotide usage frequency correlation index (TETRA) values against the most similar species in this analysis, Pseudomonas sp.NZ CP024478.1 (strain HLS-6), was 0.983, down from 0.99. On the other hand, in the phylogenetic analysis and species delimitation based on multilocus sequence analysis (MLSA) of the selected genes (16S rRNA, gyrB, rpoB rpoD), the highest similarity estimate is 91.52% with respect to Pseudomonas japonica, which is the most similar species in this analysis; values below 97% indicate that it is a new species. The phylogeny of single nucleotide polymorphisms (SNPs) in the set of genes shared by all Pseudomonas genomes (core genome) revealed that its core genome consists of 585 gene families, representing only 0.05% of the total gene family content of this group, a value that reinforces its identity as a new species. Finally, phylogeny based on the presence or absence of homologous gene families in the pan-genome yields very similar results to those obtained from SNP analysis. Tables 2 and 3 in the section method of realisation show all the results of similarity between the new species and those most similar to it.

Therefore, based on the polyphasic approach, a new species has been described for which the name Pseudomonas palmensis sp. nov (CECT Deposit No. 030222) has been proposed. The physiological characteristics and genetic analysis of this strain allow it to be unequivocally identified and differentiated from other species of the genus Pseudomonas.

The Pseudomonas palmensis strain is characterised by its beneficial effects on plants: it induces the metabolism involved in the elimination of free radicals produced in stressful situations, improving the plant's adaptation to these conditions through a mechanism of homeostasis of redox metabolism and an increase in water potential under these conditions, a clear indicator of the capacity of this strain to induce a water balance, resulting in a greater capacity of the plant to resist water stress conditions. On the other hand, it is able to improve the ferric nutrition of the plants, also improving their growth.

Being a new species, there are no patents for Pseudomonas palmensis on any of the reported effects. With respect to the genus Pseudomonas there are several publications describing their ability to produce siderophores and modulate metabolism involved in oxidative stress (Bar-Ness et. al., 1991, Journal Iron nutrition and interactions in plants, 271-281; Weger et. al., 1988, DOI: 10.1128/jb.170.10.4693-4698.1988; Ghosh et. al., 2018, DOl: https://doi.org/10.1007/s13213-018-1366-7; Vives-Peris et. al., 2018, DOI: https://doi.org/10.1007/s00299-018-2328-z). Regarding the Pseudomonas strains that are most similar to the proposed new species based on the above criteria, which are P.alkylphenolica, P.japonica or Pseudomonas sp.NZ CP024478.1 (strain HLS-6), there is no patent or publication on these species in the field of application of the present invention. There is some information on P.japonica related to industrial oil degradation (Pseudomonas japonica sp. nov., a novel species that assimilates straight chain alkylphenols. JOURNAL OF GENERAL AND APPLIED MICROBIOLOGY 2008 54;1 Strain Number cited in this paper: TISTR 1526) and with the ability to prevent adhesion of molluscs to any surface (WO2014149324A1), but not related to the agri-food field to which the present invention refers.

### THE INVENTION. -

The object of the invention described herein and which, in view of the prior art, is understood to meet the conditions of novelty and inventive step necessary to be worthy of patent rights, is the isolation and characterisation of the bacterial strain Pseudomonas palmensis BBB001 (CECT 30222), which is a microorganism belonging to the group of Gram - bacteria, genus Pseudomonas, with the capacity to stimulate the metabolism of adaptation to abiotic stress in plants, reducing oxidative stress by inducing the metabolism involved in the elimination of free radicals produced in stressful situations, improving the adaptation of the plant to these conditions through the homeostasis of the redox metabolism of the plant to these conditions, It reduces oxidative stress by inducing the metabolism involved in the elimination of free radicals produced in stressful situations, improving the adaptation of the plant to these conditions through the homeostasis of the redox metabolism, in such a way that growth and production are stimulated, as well as having an application for the mobilisation of nutrients, especially for iron nutrition.

The physiological characteristics and the genetic analysis of this strain, with a genome sequenced according to the WIPO ST.25 standard, which is integrated at the end of this descriptive report, allow it to be unequivocally identified, differentiating it from other species of the genus Pseudomonas.

In a bacterial screening of the rhizosphere of Nicotiana glauca, a strain belonging to the genus Pseudomonas was isolated and genetic analysis did not allow it to be placed in any of the known species of this genus.

Once isolated, a characterisation of the unique gene repertoire of Pseudomonas BBB001 was performed. In the comparative analysis of the 254 complete genomes, the BPGA programme identified a total of 489 genes uniquely present in Pseudomonas BBB001, of which 41 genes were associated with a specific subsystem. Among the 41 genes unique to Pseudomonas BBB001, those related to iron uptake and metabolism, and those related to the synthesis of amino acids and derivatives stand out due to their abundance. These 41 genes corresponding to SEQ ID NO: 1 to 41 of the sequence list have the capacity to encode formulations for the production of substances or compounds on an industrial scale, and can be used for heterologous expression to obtain products containing biological material from the strain.

After their characterisation, several tests were carried out to reveal biochemical activities indicative of their potential capacity to induce adaptive metabolism, reducing oxidative stress and promoting plant growth, on the one hand, and their capacity to improve ferric nutrition, on the other. These were auxin production, degradation of 1-aminocyclopropane-1-carboxylate, phosphate solubilisation and production of siderophores and chitinases, resulting positive for auxin and siderophore production in vitro. BIOLOG Eco's profile indicates that it is able to degrade the tricarboxylic acids malic, hydroxybutyric and glucosamine acid, and the degradation of 1P-glucose, cellobiose, lactose and N-acetylglucosamine stand out among the sugars. As nitrogen sources, it uses putrescine, phenylethylamine, serine and L-glycol-L-glutamic acid.

So far, experiments have been carried out involving the direct inoculation of Pseudomonas palmensis in model plants (Arabidopsis thaliana), characterising the metabolic profile of enzymes related to the control of oxidative stress and plant redox balance and the modification of photosynthetic parameters related to fluorescence as an indicator of the potential for stimulating plant growth and production, and also the state of health of the plant as a result of the effects on the plant's redox balance.

In addition, direct inoculations have been carried out on blackberry plants (Rubus sp. Var Lochness), growing in production greenhouses. The total accumulated production has been determined, and the metabolic profile of enzymes related to the control of oxidative stress in blackberry leaves has been determined at two moments of the cycle (flowering and full production), with two applications per month, during the 6 months of the production cycle.

These two experiments carried out on different plant species demonstrate that the bacterium is capable of modulating the metabolism involved in plant adaptation to abiotic stress, controlling oxidative stress by increasing the capacity to eliminate oxygen free radicals (ROS) through: 1) an increase in the activities of ROS scavenging enzymes, superoxide dismutase (SOD) or catalase (CAT), and 2) an increase in the dissipation of excess photosynthetic energy responsible for the generation of ROS free radicals (non photochemical quenching, NPQ) under normal conditions.

Another experiment has been conducted to demonstrate the ability to stimulate adaptive metabolism to salinity stress conditions in tomato plants. The strain induced a substantial improvement in water potential with respect to the control, slightly increasing the proline content, a compatible osmolyte. The increase in water potential under these conditions is a clear indicator of the ability of this strain to induce water balance, resulting in an increased ability of the plant to withstand salinity or water stress conditions.

In another experiment carried out on olive trees in production in an intensive and irrigated system, an experiment with BBB001 has been conducted to evaluate the ability to increase production under normal irrigation conditions and with a 25% reduction in irrigation. In a pre-harvest sampling, we found an increase of more than 40% in the average fresh weight of the fruit compared to the control under standard irrigation conditions and with water reduction. On the other hand, the fruit weight of the plants treated with BBB001 at root level reached the same values in both irrigation regimes.

On the other hand, experiments have been carried out on the reversion of iron deficiency chlorosis in tomato plants. These experiments were carried out by direct inoculation of Pseudomonas palmensis in tomato plants in which iron deficiency had been induced and the substrate had been adjusted to a basic pH to ensure the insolubilisation of Fe. We also tested the strain grown in a specific siderophore production medium (modified succinate medium), with the strain, and also tested only the metabolic liquid. Similar results were found with all treatments, highlighting the effect on the improvement of iron, phosphorus and potassium content, the improvement of photosynthesis, an increase in photosynthetic pigments and, therefore, an improvement in growth (increase in dry weight).

The experiments on the use of Pseudomonas palmensis BBB001 with the capacity to stimulate the metabolism of adaptation to abiotic stress in plants, reducing oxidative stress by increasing the activity of enzymes related to ROS scavenging, in such a way that growth and production are stimulated, as well as having an application as a biofertiliser because it stimulates the mobilisation of nutrients, specifically the nutrition of iron, phosphorus and potassium, are set out at the end of this report, in the section on how it is carried out.

The ultimate aim of this invention, which constitutes the technical advantage of the same, is to have a bacterium (biostimulant/biofertiliser) that improves the capacity to stimulate the metabolism of adaptation to abiotic stress in plants, reducing oxidative stress, in such a way as to stimulate growth and production. On the other hand, it has application as a biofertiliser because it stimulates the mobilisation of nutrients, specifically the nutrition of iron, phosphorus and potassium, by bringing the bacteria or any of its parts into contact with the plant by any available means, improving the effect with certain bacterial species.

Consequently, the present patent application claims the use of the strain Pseudomonas palmensis BBB001, or of the metabolites of the strain's culture broth, for its application in any type of plant species, in order to increase the plants' capacity to adapt to any abiotic stress condition, such as salinity conditions, increasing production, as well as to improve the plants' iron, phosphorus and potassium mineral nutrition.

Such use of Pseudomonas palmensis BBB001 may be as part of any preparation with the strain, either individually or in combination with other bacterial species or organisms, and by any available means that brings the bacteria into contact with the seed, root or aerial system of plants.

In particular the use of Pseudomonas palmensis BBB001 is suitable in combination with Pseudomonas fluorescens CECT 9015, or as part of any preparation with this other strain, to improve the response to salt stress; and also in combination with Arthrobacter oxydans CECT code 30231, to improve mineral nutrition in iron, nitrogen, phosphorus and potassium, both in organic and conventional farming.

### METHOD OF REALISATION.-

The strain of the genus Pseudomonas described here was isolated by studying the rhizosphere of natural populations of Nicotiana glauca in a transect on the island of Las Palmas de Gran Canaria (Canary Islands). This plant species (Nicotiana glauca) was selected because it belongs to the Solanaceae family, because it has an active secondary metabolism and because it has proved to be a good source of microorganisms with biological activity of interest in agriculture.

Rhizosphere sampling for bacterial strain isolation was carried out on natural populations of Nicotiana glauca in December 2017. As a result of this sampling, 450 strains were collected, among which Pseudomonas palmensis (BBB001, internal laboratory code) was found. The isolation of this strain was carried out on nutrient agar (PCA).

In the laboratory, this microorganism is maintained with a high survival rate in 20% glycerol in nutrient broth (Pronadisa) at -80°C or in 15% glycerol in water at -20°C and is easily recovered in the culture medium used for isolation both in solid and liquid phase at 28°C.

### I. Morphological, biochemical and genetic characteristics of Pseudomonas palmensis BBB001.-

For the characterisation of the strain, different phenotypic characters were considered and are detailed in this report: (i) colony morphology, (ii) cell morphology, (iii) metabolic profile, (iv) comparative analysis of the genome of strain Pseudomonas BBB001 and the complete genomes available for the genus Pseudomonas.

Table 1 specifies the colony morphology after 24 h incubation at 28°C on standard agar (PCA).

**TABLE 1**

| | BBB001 |
|---|---|
| Size of the colony | < 1 mm∅ |
| Shape | Circular |
| Edge | Smooth |
| Transparency | No |
| Consistency | Creamy |
| Colour | Yellow |

Growing in liquid environment (Pronadisa nutrient broth) the colour of this environment changes to yellow from the exponential phase of growth to the stationary phase of growth.

The morphological characters of Pseudomonas palmensis BBB001 after 24 h incubation at 28° on standard agar (PCA) correspond to a Gram-negative bacillus.

Once isolated and characterised, with internal reference code BBB001, several tests were carried out to demonstrate the potential of this bacterium. These were auxin production, degradation of 1-aminocyclopropane-1-carboxylate, phosphate solubilisation and production of siderophores and chitinases, with positive results for siderophore production.

Metabolic profiling (BIOLOG Eco, www.biolog.com/products-portfolio-overview/microbial-community-analysis-with-ecoplates/) indicates that it is able to degrade tricarboxylic acids: malic, hydroxybutyric and glucosaminic acid, and the degradation of 1P-glucose, cellobiose, lactose and N-acetylglucosamine stand out among the sugars. As nitrogen sources, it uses putrescine, phenylethylamine, serine and L-glycol-L-glutamic acid.

To investigate the phylogenetic relationship of Pseudomonas palmensis strain BBB001 with respect to the other species of the genus Pseudomonas and to define its taxonomic assignment, its complete genome was compared with the 254 Pseudomonas genomes that are available and complete in databases. Three main strategies were used, according to the following defining criteria for a new bacterial species:
1. Estimates of the degree of similarity between genomes: average nucleotide identity (ANI), average amino acid identity (AAI), in silico DNA-DNA hybridisation (dDDH), difference in guanine-cytosine content (G+C) and frequency of tetranucleotide usage (TETRA):
   - ANI and AAI values represent a robust measure of the evolutionary distance between two genomes; when these values are less than 95%, a new species is assumed. It has been empirically proven that ANI and AAI values of 95-96 % are equivalent to a DDH (DNA-DNA hybridisation) value of 70 %, another parameter commonly used to delimit prokaryotic species (e.g., Konstantinidis and Tiedje 2005, PNAS 102:2567-2572; Richter and Rosselló-Mora 2009, PNAS 106:19126-19131; Kim et al.2014, International Journal of Systematic and Evolutionary Microbiology 64:346-351).
   - Differences within-species in G+C content are generally no more than 1 %; Meier-Koltho et al. 2014, International Journal of Systematic and Evolutionary Microbiology 64:352-356).
   - The dDDH index has shown a higher correlation with traditional DDH values (Auch et al. 2010, Standards in Genomic Sciences 2:1). Therefore, dDDH values higher than 70 % are considered to be indicative that the genomes compared belong to the same species.
   - TETRA values between the genome of Pseudomonas BBB001 and the 26 most closely related genomes. Genomes of closely related strains are expected to show similar tetranucleotide usage frequencies, with correlation indices (TETRA) equal to or greater than 0.99, so TETRA values below 0.99 are indicative of a new species (Teeling et al. 2004, Environmental Microbiology 6:938947; Richter and Rosselló-Mora 2009, PNAS 106:19126-19131).
2. Phylogenetic analysis and species delimitation based on multilocus sequence analysis (MLSA):
   - Sequencing of the 16S ribosomal gene (16S rRNA) is the basic tool in current bacterial species classification systems. It is generally accepted that two different species will have similarity values for the 16S rRNA gene of less than 98.65-99 % (Kim et al. 2014, International Journal of Systematic and Evolutionary Microbiology 64:346-351). However, in many cases, the 16S rRNA gene may offer limited resolving power. On the one hand, this gene may have a low ability to discriminate between closely related species. On the other hand, some species may show very similar 16S rRNA sequences (<99%), despite being clearly different based on DDH values (Ash et al. 1991, International Journal of Systematic and Evolutionary Microbiology 41:343-346; Rosselló-Mora and Amann 2001, FEMS Microbiology Reviews 25:39- 67). In the specific case of the genus Pseudomonas, Mulet et al. have recommended the analysis of three additional genes (gyrB, rpoB and rpoD) to delimit species, identify new strains, or resolve phylogenetic relationships within this group (Mulet et al. 2012a, Systematic and Applied Microbiology 35:455-464; Mulet et al. 2012b, Systematic and Applied Microbiology 35:145-149; Sánchez et al. 2014, Systematic and Applied Microbiology 37:89-94). Mulet et al. established an average similarity of 97 % as a criterion for species delimitation based on the MLSA of these four genes within the genus Pseudomonas (Mulet et al. 2010, Environmental Microbiology 12:1513-1530).
3. Phylogenetic analysis at the whole genome level, based on the identification of variants (single nucleotide polymorphisms, SNPs) in the conserved fraction of the genome (core genome) and on the presence/absence of genes in the pan-genome:
   - Phylogeny of SNPs (single nucleotide polymorphism) in the set of genes shared by all Pseudomonas genomes (core genome), and phylogeny based on the presence or absence of homologous gene families in the pan-genome. Advances in sequencing and comparison of bacterial genomes have revealed unexpected variability in the gene content of different species within a genus, and even of different strains of the same species. The genomes of Escherichia coli strains, for example, share only 40% of their genes (Rasko et al. 2008, Journal of Bacteriology 190:6881-6893). This has led to the formulation of the concept of the core genome to define the set of genes shared by all bacterial species in a given group. Genes that are absent in one or more members of the group, and therefore do not form part of the core genome, are called accessory genes. Finally, the complete gene repertoire (i.e. the sum of the core genome and accessory genes) has been termed the pan-genome.

Finally, as a result of the Pseudomonas pan-genome analysis, the repertoire of unique genes present in Pseudomonas strain BBB001 was characterised.

### 1. Estimates of the degree of similarity between genomes.-

### 1.1. Parameters average nucleotide identity (ANI) and average amino acid identity (AAI):

The first step of this analysis consists in the comparison at nucleotide and amino acid level of the genome of Pseudomonas BBB001 with all the complete genomes available in the RefSeq database (NCBI Reference Sequence database) for the genus Pseudomonas.

Estimates of nucleotide identity indices (NIA) were calculated with the OAU.jar program.

The tool Compared v0.0.23 (https://github.com/dparks1134/CompareM) was used for the comparison of amino acid content (AAI). This tool identifies homologous genes and calculates the identity between them.

### 1.2. DNA-DNA hybridisation in silico (dDDH) and G+C content:

Based on the results of the previous section, the 26 genomes located in the fourth quartile of the distribution of ANI values (i.e., predictably, those most closely related to Pseudomonas BBB001) were selected to calculate intergenomic distances and dDDH indices, using the web server GGDC (Genome-to-Genome Distance Calculator). This server also calculates the difference in G+C content of the analysed genomes.

### 1.3. Tetranucleotide usage frequencies (TETRA):

JSpecies software (Richter and Rossellò-Mora 2009, PNAS 106:19126-19131) was used to calculate differences in tetranucleotide usage profiles between the Pseudomonas BBB001 genome and the 26 most closely related genomes.

### 2. Phylogenetic analysis and species delimitation based on multilocus sequence analysis (MLSA).-

In the multilocus analysis, also performed on the subset of 26 genomes, the four housekeeping genes recommended for species delimitation in the genus Pseudomonas were included: 16S, gyrB, rpoD and rpoB (e.g., Mulet et al. 2012a, Systematic and Applied Microbiology 35:455-464).
- 16S: A home-made bash scripting pipeline was used to extract the multiple copies of this gene in each of the whole genomes analysed. These copies were collapsed into a consensus in which each variable position was represented as an ambiguity.
- gyrB, rpoD and rpoB: The gyrB, rpoD and rpoB genes were extracted from the whole genomes in question by mapping against reference using BWA 0.7.12 (Li and Durbin 2010, Bioinformatics 26:589- 595).

In addition, in order to compare the resulting phylogeny with that obtained by Mulet et al. when investigating the phylogenetic position of Pseudomonas alkylphenolica (Mulet et al. 2015, International Journal of Systematic and Evolutionary Microbiology 65:4013-4018), the sequences of 13 other species or strains used in that work, without complete published genomes, were included in the analysis.

For each of the above genes, an alignment of all sequences was performed using the Mafft v7.310 program (Katoh et al. 2002, Nucleic Acids Research 30:3059-3066). In each of the 4 alignments, regions that were ambiguous (i.e. regions missing in some sequences and/or misaligned) were removed.

Finally, the 4 genes were concatenated and the best-fitting nucleotide substitution models were estimated for each gene using Partition Finder 2 (Lanfear et al. 2012, Molecular Biology and Evolution 29:1695-1701). MrBayes v3.2 (Ronquist et al. 2012, Systematic Biology 61:539-542) was used to obtain the phylogenetic tree.

A study of the similarity of these 4 genes between Pseudomonas BBB001 and the rest of the species or strains was also carried out using a Biopython module called Distance Calculator. In the case of the 16S gene, as it is a multiple copy gene, the average similarity obtained for each copy was calculated.

### 3. Phylogenetic analysis based on whole genome sequences.-

BPGA software (Chaudhari et al. 2016, Scientic Reports 6:24373) was used to perform homology clustering of the annotated genes in the 253 complete Pseudomonas genomes and in Pseudomonas BBB001. The clustering threshold was 50% homology, thus clustering homologous genes as well as genes that may belong to the same family. BPGA identifies gene families shared by all the genomes analysed (core genome), accessory genes and, in addition, unique functions, i.e. genes that have not been grouped in any of the clusters created.

### 3.1. Phylogeny of SNPs (single nucleotide polymorphisms) present in core genes:

First, a homemade bash scripting pipeline was designed to extract the nucleotide sequences associated with the genes identified as core genome by BPGA.

The genetic distance of these sequences was analysed using DistanceCalculator and an alignment was generated for each of the core genes using Mafft v7.310 (Katoh et al. 2002, Nucleic Acids Research 30:3059-3066). Finally, gap content information was extracted from the generated alignments to identify poor quality alignments.

For the study of SNPs, it is necessary to ensure that all the positions extracted are homologous positions, so those genes that included sequences with a distance greater than 50% and that also included a gap content greater than 10% of the sequence length were eliminated.

Geneious v10.2.2 (Kearse et al. 2012, Bioinformatics 28:1647-1649) was used to remove positions where more than 10% of the sequences had a gap and finally extract the SNPs in PHYLIP format.

RAxML v8.2.9 software (Stamatakis 2014, Bioinformatics 30:1312-1313) was used to generate the maximum likelihood phylogenetic tree of all concatenated SNPs. The support for each node was calculated by performing 1000 bootstrap replicates.

### 3.2 Phylogeny of the matrix presence/absence of genes or gene families in the pan-genome:

The gene presence/absence matrix produced by BPGA was used to reconstruct a maximum likelihood phylogenetic tree using IQTREE (Chernomor et al. 2016, Systematic Biology 65:997-1008). Support for each node was calculated by performing 1000 bootstrap replicates.

### 4. Characterisation of the unique gene repertoire of Pseudomonas BBB001.

To determine whether the unique functions identified had not been previously described, a local database of the nr (non-redundant) protein database available in the NCBI/EMBL/DDBJ database collection was created to search for them using the BLAST+ 2.6.0 program (Camacho et al. 2008, BMC Bioinformatics 10:421).

A home-made pipeline of scripts written in bash language was used to extract the relevant information from the first match obtained in the search performed. The unique genes are presented as an attachment.

### 5. Discussion of the results.-

The results obtained by comparing our strain with 254 Pseudomonas genomes and which have allowed us to identify it as a new species, Pseudomonas palmensis BBB001, are given below; all values are the result of comparison with the most similar species available. Thus its unique genetic characteristics are: with respect to Pseudomonas alkylphenolica, ANI and AAI values of 83.39% and 85.11%, respectively (table 2), estimated dDDH values of 27.10 %, G+C content, which shows a difference of 3.43 % (table 2). It also shows TETRA values of 0.983, compared to the most similar species in this analysis Pseudomonas sp.NZ CP024478.1 (strain HLS-6) (table 2).

Table 2 shows the results of the estimates of the degree of similarity between genomes. For each genome, its length (in base pairs, bp), the number of annotated genes, and the values of ANI, AAI, dDDH, intergenomic distance, difference in G+C content and TETRA index estimated in the comparison with the genome of Pseudomonas BBB001 are specified. In column 1, after the species name, the RefSeq genome accession number is given.

**TABLE 2**

| **Compared Genomes** | **Gene length (pb)** | **Annota ted genes** | **ANI (%)** | **AAI (%)** | **DDH (%)** | **Intergeno mic distance** | **Diference s G+C (%)** | **TETRA Index** |
|---|---|---|---|---|---|---|---|---|
| *New species indicator value* | | | <95% | <95% | <70% | | 1% | <0.99 |
| *Pseudomonas alkylphenolica* NZ CP009048 1 | 5764623 | 5080 | 83.389 | 85.110 | 27.100 | 0.160 | 3.430 | 0.938 |
| *Pseudomonas* sp NZ CP007012 1 | 5891313 | 5129 | 82.128 | 81.200 | 26.100 | 0.167 | 0.780 | 0.978 |
| *Pseudomonas monteilii* NC 023075 1 | 6000088 | 5315 | 82.126 | 81.210 | 25.700 | 0.169 | 1.600 | 0.974 |
| *Pseudomonas monteilii* NC 023076 1 | 5945121 | 5250 | 81.993 | 81.290 | 25.700 | 0.169 | 1.570 | 0.974 |
| *Pseudomonas putida* NC 0157331 | 5984791 | 5263 | 81.864 | 80.810 | 25.500 | 0.171 | 1.740 | 0.975 |
| *Pseudomonas plecoglossicida* NZ CP010359 1 | 6233255 | 5533 | 81.871 | 81.240 | 25.500 | 0.170 | 1.660 | 0.975 |
| *Pseudomonas putida* NC 0199051 | 5875751 | 5344 | 81.843 | 81.000 | 25.400 | 0.171 | 1.460 | 0.977 |
| *Pseudomonas mosselii* NZ CP023299 1 | 5751089 | 5029 | 81.11 | 81.170 | 25.400 | 0.171 | 0.290 | 0.981 |
| *Pseudomonas mosselii* NZ CP024159 1 | 5742166 | 5045 | 81.144 | 81.140 | 25.400 | 0.172 | 0.360 | 0.981 |
| *Pseudomonas entomophila* NC 008027 1 | 5888781 | 5055 | 81.903 | 81.010 | 25.200 | 0.173 | 0.100 | 0.982 |
| *Pseudomonas* sp NZ CP024478 1 | 5276695 | 4704 | 81.961 | 84.040 | 25.200 | 0.173 | 4.180 | 0.892 |
| *Pseudomonas putida* NC 021491 1 | 6870828 | 6227 | 81.515 | 81.200 | 25.100 | 0.173 | 2.460 | 0.968 |
| *Pseudomonas* sp NZ LN847264 1 | 6012948 | 5279 | 82.057 | 81.000 | 25.000 | 0.174 | 0.130 | 0.983 |
| *Pseudomonas putida* NC 021505 1 | 6156702 | 5390 | 81.508 | 80.980 | 24.900 | 0.175 | 1.730 | 0.969 |
| *Pseudomonas* putida NC 009512 1 | 5959965 | 5200 | 81.239 | 80.930 | 24.800 | 0.176 | 2.200 | 0.959 |
| *Pseudomonas putida* NC 017986 1 | 6085450 | 5538 | 81.306 | 80.750 | 24.800 | 0.176 | 2.290 | 0.959 |
| *Pseudomonas putida* NC 002947 4 | 6181874 | 5564 | 81.066 | 80.570 | 24.700 | 0.177 | 2.530 | 0.963 |
| *Pseudomonas putida* NC 017530 1 | 5731542 | 5109 | 81.121 | 80.950 | 24.700 | 0.176 | 2.320 | 0.959 |
| *Pseudomonas* sp NZ CP011525 1 | 6178826 | 5399 | 81.233 | 80.620 | 24.700 | 0.176 | 2.720 | 0.961 |
| *Pseudomonas putida* NC 010322 1 | 6078431 | 5398 | 81.221 | 80.950 | 24.600 | 0.177 | 2.120 | 0.969 |
| *Pseudomonas mosselii* NZ CP009365 1 | 6247861 | 5563 | 81.569 | 80.540 | 24.600 | 0.177 | 0.670 | 0.982 |
| *Pseudomonas putida* NC 010501 1 | 5774331 | 5193 | 81.013 | 81.220 | 24.300 | 0.179 | 2.620 | 0.959 |
| *Pseudomonas* sp NC 022738 1 | 5644570 | 5411 | 80.845 | 81.030 | 24.000 | 0.182 | 2.230 | 0.961 |
| *Pseudomonas parafulva* NZ CP009747 1 | 5087620 | 4295 | 80.865 | 80.400 | 23.900 | 0.183 | 0.590 | 0.964 |
| *Pseudomonas* sp NZ LN865164 1 | 4835346 | 4180 | 79.681 | 79.980 | 23.300 | 0.188 | 2.380 | 0.942 |
| *Pseudomonas parafulva* NZ CP019952 1 | 4690484 | 4055 | 79.923 | 80.080 | 23.200 | 0.188 | 2.350 | 0.942 |

The similarity estimates in the MLSA analysis for the 16S rRNA gene (sequence deposited in Genebank MW009702) yielded values between 98.52 % and 99.42 %, close to or above the threshold value to be included in the same species, however, the similarity values for the other three genes included in the MLSA are, however, considerably lower <97% (91. 52%, with respect to Pseudomonas japonica, the most similar species in this analysis, Table 3) established by Mulet et al. for species delimitation based on the MLSA of these four genes within the genus Pseudomonas.

Table 3 shows the percentage of similarity for each of the four genes included in the MLSA and the average similarity calculated for the four genes together.

**TABLE 3**

| **Compared Genomes** | **16S** | **gyrB** | **rpoB** | **rpoD** | **Average Similitude** |
|---|---|---|---|---|---|
| *Pseudomonas japonica* (AB126621.2,GQ996725.1,HE577800.1,HE577795.1) | 98.81 | 89.65 | 94.04 | 83.59 | 91.52 |
| *Pseudomonas alkylphenolica* NZ CP009048.1 | 98.95 | 90.49 | 94.04 | 82.57 | 91.51 |
| Pseudomonas sp NZ CP024478.1 | 99.42 | 88.67 | 91.72 | 83.93 | 90.93 |
| *Pseudomonas vranovensis* (AY970951.1,HE577791.1,HE577799.1,HE577793.1) | 98.52 | 90.63 | 93.16 | 80.37 | 90.67 |
| *Pseudomonas* sp NZ CP007012.1 | 98.54 | 88.67 | 92.60 | 79.53 | 89.84 |
| *Pseudomonas mosselii* (AF0726882,FN554207.1,FN554744.1,FN554491.1) | 98.24 | 87.41 | 93.16 | 80.37 | 89.80 |
| *Pseudomonas mosselii* NZ CP024159.1 | 98.43 | 86.99 | 93.27 | 80.37 | 89.77 |
| *Pseudomonas mosselii* NZ CP023299.1 | 98.42 | 86.99 | 93.27 | 80.37 | 89.76 |
| *Pseudomonas plecoglossicida* NZ CP010359.1 | 98.63 | 87.83 | 92.49 | 78.00 | 89.24 |
| *Pseudomonas monteilii* NC 023075.1 | 98.62 | 87.83 | 92.49 | 78.00 | 89.24 |
| *Pseudomonas monteilii* NC 023076.1 | 98.62 | 87.83 | 92.49 | 78.00 | 89.24 |
| *Pseudomonas putida* NC 019905.1 | 98.41 | 88.11 | 92.27 | 77.83 | 89.16 |
| *Pseudomonas monteilii* (AF064458.1,FN554205.1,AJ717455.1,FN554488.1) | 98.38 | 87.55 | 92.05 | 78.34 | 89.08 |
| *Pseudomonas putida* NC 015733.1 | 97.83 | 87.83 | 92.49 | 78.00 | 89.04 |
| *Pseudomonas plecoglossicida* (AB009457.1, FN554218.1,AJ717456.1, FN554503.1) | 98.52 | 87.13 | 92.27 | 78.00 | 88.98 |
| *Pseudomonas guariconensis* (HF674459.1,LN851841.1,LN851843.1,LN851842.1) | 97.68 | 87.55 | 92.16 | 78.51 | 88.98 |
| *Pseudomonas putida* NC 021505.1 | 98.55 | 86.85 | 92.72 | 77.16 | 88.82 |
| *Pseudomonas putida* NC 010322.1 | 98.48 | 87.69 | 91.94 | 77.16 | 88.82 |
| *Pseudomonas putida* NC 002947.4 | 98.38 | 86.71 | 92.60 | 77.50 | 88.80 |
| *Pseudomonas* sp NZ LN847264.1 | 98.36 | 85.59 | 92.27 | 78.68 | 88.73 |
| *Pseudomonas putida* NC 010501.1 | 98.19 | 87.69 | 91.83 | 76.99 | 88.67 |
| *Pseudomonas putida* (D84020.1,AB039451.1,AJ717474.1,AB039581.1) | 97.96 | 86.85 | 92.27 | 77.16 | 88.56 |
| *Pseudomonas putida* NC 017530.1 | 98.40 | 86.57 | 92.38 | 76.82 | 88.54 |
| *Pseudomonas* sp NC 022738.1 | 98.62 | 85.87 | 92.38 | 76.82 | 88.42 |
| *Pseudomonas entomophila* NC 008027.1 | 98.41 | 85.87 | 91.72 | 77.66 | 88.42 |
| *Pseudomonas putida* NC 021491.1 | 98.41 | 85.45 | 92.05 | 77.66 | 88.40 |
| *Pseudomonas taiwanensis* (EU1036292,FJ418634.1,HE577797.1,HE577796.1) | 98.52 | 85.87 | 92.16 | 76.82 | 88.35 |
| *Pseudomonas mosselii* NZ CP009365.1 | 98.01 | 84.90 | 92.16 | 78.17 | 88.31 |
| *Pseudomonas putida* NC 017986.1 | 98.49 | 86.43 | 91.94 | 75.97 | 88.21 |
| *Pseudomonas soli* (HF930598.1,LN851840.1,HF930596.1,HF930597.1) | 97.89 | 85.03 | 92.05 | 77.83 | 88.20 |
| *Pseudomonas* sp NZ CP011525.1 | 98.40 | 86.85 | 91.94 | 75.47 | 88.17 |
| *Pseudomonas putida* NC 009512.1 | 98.47 | 86.43 | 91.61 | 75.97 | 88.12 |
| *Pseudomonas fulva* (AB060136.1,AB039395.1,AJ717419.1,AB039586.1) | 97.82 | 85.87 | 90.73 | 78.00 | 88.11 |
| *Pseudomonas parafulva* (AB060132.1,FN554216.1,AJ717471.1,FN554500.1) | 97.82 | 86.01 | 90.84 | 77.33 | 88.00 |
| *Pseudomonas* sp NZ LN865164.1 | 97.88 | 85.31 | 90.40 | 77.83 | 87.86 |
| *Pseudomonas cremoricolorata* (AB060137.1,FN554181.1,AJ717476.1,FN554462.1) | 98.24 | 84.76 | 90.29 | 76.99 | 87.57 |
| *Pseudomonas parafulva* NZ CP019952.1 | 97.84 | 83.92 | 90.29 | 77.66 | 87.43 |
| *Pseudomonas parafulva* NZ CP009747.1 | 93.89 | 87.13 | 90.29 | 78.00 | 87.33 |
| *Pseudomonas aeruginosa* (X06684.1,AB039386.1,AJ717442.1,AB039607.1) | 93.61 | 84.06 | 89.29 | 73.60 | 85.14 |

Phylogeny of SNPs in the set of genes shared by all Pseudomonas genomes (core genome). Comparison of the 254 complete genomes available for the genus Pseudomonas and the genome of Pseudomonas BBB001, performed with BPGA software, revealed that its core genome consists of 585 gene families, representing only 0.05 % of the total gene family content of this group. The vast majority of the core genome families are associated with functions of translation and ribosome biogenesis, or amino acid metabolism and transport.

After extracting the orthologous genes (275 genes) from the group of core genome families, a total of 164394 polymorphic positions (single nucleotide polymorphisms, SNPs) were identified in the 254 genomes. Based on the SNPs, five major distinct groups are obtained, which are broadly consistent with the phylogeny of the genus Pseudomonas presented in Gomila et al. 2015 (Frontiers in Microbiology 6:214):
- Group of *P. aeruginosa, P. denitrificans, P. knackmussii* y *P. citronellol is.* (102 genomes)
- Group of *P. stutzeri,* to which it also belongs *P. balearica.* (11 genomes)
- Group of *P. syringae,* which also includes *P. amygdali, P. savastanoi, P. viridiava* y *P. cichorii.* (22 genomes)
- Group of *P. fluorescens,* which includes other species such as P. mandelii, P. koreensisis, P. brassicacearum, *P. protegens* o *P. fragi.* (57 genomes)
- Group of *P. putida,* within which you can find *Pseudomonas* BBB001. (43 genomes)
- Other groups: (19 genomes)

The P. putida group includes a total of 43 of the 254 complete genomes analysed and available in databases. In addition to all genomes belonging to P. putida, other species such as P. monteilii or P. parafulva are found in this group. In agreement with the MLSA results, Pseudomonas BBB001 forms a statistically well-supported subgroup with the species P. alkylphenolica and Pseudomonas sp. NZ CP024478.1 (strain HLS-6). Within this subgroup it is not possible to make a direct comparison with the MLSA results, as the species P. japonica and P. vranovensis do not have complete published genomes and are therefore not included in the core genome analysis. However, while the MLSA phylogeny did not resolve the phylogenetic position of Pseudomonas BBB001 relative to the other species, the SNP phylogeny provided greater resolution, recovering Pseudomonas BBB001 as the basal lineage of this subgroup (i.e. Pseudomonas BBB001 was the first to separate from P. alkylphenolica and Pseudomonas sp. strain HLS-6).

Phylogeny based on the presence or absence of homologous gene families in the pan-genome. Comparative analysis of the 254 genomes revealed the presence of a total of 65137 accessory gene families in the pan-genome. Information on the presence or absence of genes in each genome was transformed into a binary matrix.

In general, the phylogeny obtained from the information on the presence/absence of genes in the pan-genome was similar to that obtained based on SNP analysis, although some significant differences were observed. For example, the P. stutzeri group, which according to the SNP phylogeny is closer to the P. putida group than P. aeruginosa, appears as the basal group, the most divergent of all those analysed, based on its accessory gene content.

In the pan-genome phylogeny, Pseudomonas BBB001 is also part of the P. putida group, constituting a distinct subgroup together with P. alkylphenolica and Pseudomonas sp. NZ CP024478.1 (strain HLS-6). In this case, in contrast to the SNP phylogeny, the analysis establishes, with a significant support value, a closer relationship between Pseudomonas BBB001 and Pseudomonas sp. (strain HLS-6). It is therefore likely that the accessory gene repertoire of Pseudomonas BBB001 is more similar to that of Pseudomonas sp. (strain HLS-6) than to that of P. alkylphenolica.

### II. Evidence for Pseudomonas palmensis as a stimulant of plant adaptive metabolism to abiotic stress and as an enhancer of iron nutrition. -

1°. Direct inoculation experiment of Pseudomonas palmensis on a model plant (Arabidopsis thaliana). Experiment carried out in an experimental chamber under controlled conditions of light, humidity and temperature on a total of 42 plants and an N = 3, with a randomised block model - a cell suspension of the strain was inoculated in pre-germinated A.thaliana seedlings at 2 and 5 weeks after germination. At 6 weeks fluorescence photosynthetic parameters (F0, Fv/Fm, PSII, NPQ) were measured, plants were harvested, weighed and analysed for enzymatic activities related to ROS scavenging (SOD, APX, CAT), and Ascorbate glutathione cycle (dehydroascorbate reductase - DHR, monodehydroascorbate reductase-MDHR, glutathione reductase-GR). A significant increase in energy dissipation capacity (Non photochemical quenching-NPQ) was observed and the metabolic profile of enzymes related to oxidative stress control consisted of a significant increase in catalase (CAT) and glutathione reductase (GR) activity, and a decrease in APX activity. An increase in fresh weight was recorded.
2°. Direct inoculation experiment of Pseudomonas palmensis on blackberry (Rubus var. Lochness) in production greenhouses. Experiment carried out under real field production conditions on a total of 120 plants and n=3, with a randomised block experimental model. Cell suspensions of the strain were applied at root level during the 6-month production cycle, twice a month from October to March. Total production was collected and two sampling moments, full flowering and full fruiting, were determined. At each moment, photosynthetic fluorescence parameters were measured (F0, Fv/Fm, PSII, NPQ) and leaves were collected, where photosynthetic pigments and enzymatic activities related to ROS scavenging (SOD, APX, CAT), and Ascorbate-glutathione cycle (dehydroascorbate reductase-DHR, monodehydroascorbate reductase-MDHR, glutathione reductase-GR) were analysed; antioxidant potential (ability to inhibit B-carotene oxidation) and levels of malondialdehyde-MDA, an indicator of the oxidative status of the cell, were assessed. A significant increase in non-photochemical quenching (NPQ) and a decrease in chlorophyll B were observed at both sampling times; the metabolic profile of enzymes related to oxidative stress control consisted of a significant increase in superoxide dismutase (SOD) and monodehydroascorbate reductase (MDHA) activity, and a decrease in ascorbate peroxidase (APX) activity, associated with an improvement in antioxidant potential and a slightly lower level of malondialdehyde than the control at fruit set. An increase in fruit yield was recorded.
3°. Adaptive metabolism stimulation experiment under salinity conditions in tomato (var Casillas). This experiment was carried out in a greenhouse under controlled conditions of light, humidity and temperature on a total of 336 plants, 4 treatments, 3R per treatment, n=28, organised in randomised blocks. Tomato seeds were germinated in pots, with 3 applications of a bacterial suspension at 2, 4 and 6 weeks after germination; a 500 mM NaCl solution was applied in the irrigation for one week, normal irrigation was maintained for another week, after which the water potential was measured with a Scholander camera and the dry weight of the root, aerial part and height were determined. A significant decrease in height and aerial weight, an increase in water potential and an increase in proline, an osmolyte related to water stress resistance, were observed. In this experiment, the combination of P.palmensis with another pseudomonad P.fluorescens CECT9015 was also tested. It was observed that the combination of both produced significant increases in height and root dry weight, as well as an increase in water potential and a significant increase in proline, this effect being synergistic according to Colby (http://www.jstor.org/stable/4041058) since the CECT9015 strain individually was not responsible for these increases.
4°. An experiment was carried out on irrigated super-intensive olive trees, reducing irrigation by 25% (eliminating one irrigation in 4). Pseudomonas strain BBB001 was tested in root application. Applications were made every 15 days from April to September (12 applications). The number of plants in the trial was 20 per treatment (3) plus the control, with the two irrigation regimes (100% water and 75% water). Follow-up sampling (September) prior to harvest indicated that the BBB001 strain applied at root level caused a 44.52% increase in fresh weight of olives over the control with water (mean weight of 20 fruits, n=3), and 45.96% over the control with water reduction, reaching the same weight of olives treated with BBB001 in the two irrigation regimes.
5°. Reversal of iron chlorosis in tomato plants (var. Casillas). Experiment carried out in a greenhouse under controlled conditions of light, humidity and temperature on a total of 216 plants, 9 treatments, 24 plants per treatment organised in 3 replicates, following a randomised block experimental model. The substrate was maintained at basic pH with 5mM sodium bicarbonate buffer throughout the experiment. Seeds were germinated in substrate pots, watered with iron-free Hoagland's solution once a week for 8 weeks until chlorosis appeared. Once the chlorosis appeared, they started to be irrigated with Hoagland with Fe, providing Fe in insoluble form (CI3Fe), maintaining a control with Hoagland solution complete with soluble Fe; 3 doses of bacteria were applied with a difference of 4 days between applications. After 2 weeks, chlorosis had been reversed, fluorescence photosynthetic parameters (F0, Fv/Fm, PSII, NPQ) were measured and plants were harvested. Dry weight, photosynthetic pigments, and leaf nutrient analysis were analysed. An increase in Fo, Fv/Fm and 6PSR, and a decrease in energy dissipation (NPQ) were observed, together with an increase in photosynthetic pigments (chlorophyll a, b and carotenes) indicating an improvement in plant fitness. Nutrient analysis indicated an increase in iron concentration of 25%, as well as an improvement in zinc content, and an increase of 8.46% and 1.3% in the analysis of phosphorus and potassium uptake, respectively.

In the same experiment, we tested 1) P. palmensis grown in nutrient broth (results shown above), and 2) P. palmensis grown in siderophore production medium, 3) bacterial metabolites contained in the culture medium responsible for the mobilisation of insoluble Fe (siderophore medium). In general, the results were similar for dry weight and pigments to those indicated in the previous paragraph, with P. palmensis always standing out. All these treatments were also tested in combination with an auxin-producing strain of the species Arthrobacter oxydans Code CECT 30231. In general, the results were similar for dry weight and pigments, with P. palmensis alone always standing out; however, the combination of P. palmensis with Arthrobacter oxydans CECT Code 30231 had a synergistic effect according to Colby (http://www.jstor.org/stable/4041058) on Fe uptake, increasing the Fe content by 36.4% with respect to the control and the effect of each strain individually. An increase of 1.5%, 9.7% and 9.5% was also observed in the analysis of nitrogen, phosphorus and potassium uptake.

### INDUSTRIAL APPLICATION. -

Given the above mentioned properties of Pseudomonas palmensis BBB001 as a stimulator of adaptive metabolism to abiotic stress, improving the oxidative balance of the plant, modulating the activity of enzymes related to ROS scavenging and for its capacity to mobilise nutrients, specifically iron, nitrogen, phosphorus and potassium, this bacterial strain has a specific application in the agri-food, chemical and pharmaceutical industry, as it can be used as part of any preparation (individually or in combination with other microorganisms) and by bringing them (the strain or any part of it) into contact with the seed, the root or aerial system of plants by any available means, in any plant species, or in any form of in vitro culture, to increase the strain's capacity to adapt to abiotic stress conditions and improve mineral nutrition, specifically iron, nitrogen, phosphorus and potassium.

## Claims

1. **Bacterial strain of the species *Pseudomonas palmensis* with deposit number CECT 30222,** microorganism of the Gram- bacterial group, genus Pseudomonas, **characterized by** comprising the nucleotide sequences corresponding to SEQ ID NO.: 1 to 41.

2. Bacterial strain of the species *Pseudomonas palmensis* with deposit number CECT 30222, according to claim 1, **characterized by** its ability to stimulate the adaptive metabolism to abiotic stress in plant species, modulating the oxidative response by inducing the metabolism involved in the elimination of free radicals produced during the stress situation, improving plant growth and production.

3. Bacterial strain of the species *Pseudomonas palmensis* with deposit number CECT 30222, according to claim 2, **characterized by** its ability to increase the activity of ROS-scavenging enzymes in plant species.

4. Bacterial strain of the species *Pseudomonas palmensis* with deposit number CECT 30222, according to claim 2, **characterized by** its ability to increase the dissipation of excess photosynthetic energy responsible for the generation of ROS free radicals (NPQ) in plant species.

5. Bacterial strain of the species *Pseudomonas palmensis* with deposit number CECT 30222, according to claim 1, **characterized by** its capacity to stimulate the absorption of iron, phosphorus and potassium, improving mineral nutrition.

6. Use of the bacterial strain of the species *Pseudomonas palmensis* with deposit number CECT 30222, or of the siderophores present in the supernatant of the culture broth of the strain responsible for the mobilization of insoluble Fe, as per claims 1 and 2, for application in any type of agricultural or forestry crop, in order to improve the plant's adaptation to any abiotic stress condition.

7. Use of the bacterial strain of the species *Pseudomonas palmensis* with deposit number CECT 30222, or of the siderophores present in the supernatant of the culture broth of the strain responsible for the mobilization of insoluble Fe, as per claims 1 and 2, for application to any plant species under high salinity conditions in soil or irrigation water.

8. Use of the bacterial strain of the species *Pseudomonas palmensis* with deposit number CECT 30222, as per claims 1 and 2, for application in any plant species in order to increase the production of vegetables in plants such as *Arabidopsis thaliana,* and of plant fruits, such as blackberries, olives or tomatoes.

9. Use of the bacterial strain of the species *Pseudomonas palmensis* with deposit number CECT 30222, or of the siderophores present in the supernatant of the culture broth of the strain responsible for the mobilization of insoluble Fe, as per claims 1 and 5, for application in any plant species to improve the mineral content of iron, phosphorus and potassium.

10. Use of the bacterial strain of the species *Pseudomonas palmensis* with deposit number CECT 30222, according to claims 6 to 9, as part of any bacterial preparation, either individually or in combination with other organisms, and by any means available that puts the bacterium in contact with the seed, the root or aerial system of the plants.

11. Use of the bacterial strain of the species *Pseudomonas palmensis* with deposit number CECT 30222, according to claim 10, in combination with *Pseudomonas fluorescens* (CECT 9015), or as part of any preparation with *Pseudomonas fluorescens* (CECT 9015), to improve the response to saline stress.

12. Use of the bacterial strain of the species *Pseudomonas palmensis* with deposit number CECT 30222, according to claim 10, in combination with *Arthrobacter oxydans* (CECT 30231), or forming part of any preparation with *Arthrobacter oxydans* (CECT 30231), to improve the mineral content of iron, nitrogen, phosphorus and potassium, both in organic and conventional agriculture.
